(19) Europäisches Patentamt
European Patent Office
Office européen des brevets

(11) Publication number: 0 377 968 A2

# (12) EUROPEAN PATENT APPLICATION

(21) Application number: 89312864.5

(51) Int. Cl.5: A61K 37/02

(22) Date of filing: 11.12.89

The title of the invention has been amended (Guidelines for Examination in the EPO, A-III, 7.3).

(30) Priority: 09.12.88 JP 311717/88

(43) Date of publication of application: 18.07.90 Bulletin 90/29

(84) Designated Contracting States: AT BE CH DE ES FR GB GR IT LI LU NL SE

(71) Applicant: YAMANOUCHI PHARMACEUTICAL CO. LTD.
No. 3-11 Nihonbashi-Honcho, 2-chome
Chuo-ku
Tokyo(JP)

(72) Inventor: Itoi, Motokazu
2-6-1, Kunitachi-shi Higashi
Tokyo(JP)
Inventor: Kobayashi, Shizuko
3-25-31-906, Shakujii Nerima-ku
Tokyo(JP)
Inventor: Ishii, Yasuo
3-9-420, Shibasonomachi
Kawaguchi-shi Saitama(JP)
Inventor: Kasuya, Minako
1-61, Komukainishimachi Saiwai-ku
Kawasaki-shi Kanagawa(JP)

(74) Representative: Geering, Keith Edwin et al
REDDIE & GROSE 16 Theobalds Road
London WC1X 8PL(GB)

## (54) Glutathione esters as prophylactic and therapeutic agents against cataracts.

(57) Glutathione monoalkyl esters and salts therefore are effective as prophylatic and therapeutic agents for cataract and as radiation-protecting agents. Among glutathione monoalkyl esters, glutathione isopropyl ester is particularly preferred.

EP 0 377 968 A2

## PROPHYLACTIC AND THERAPEUTIC AGENTS FOR CATARACT

The present invention relates to prophylactic and therapeutic agents for cataract and radiation-protecting agents comprising glutathione monoalkyl esters of general formula (I)

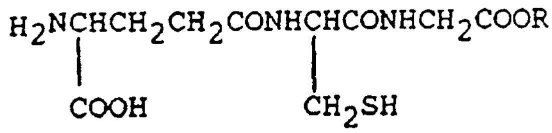

(I)

wherein

R represents an alkyl group, or salts thereof.

Cataract refers to a state in which the stromatic lens has decreased optical permeability caused by degeneration and the deposition of certain materials.

Cataract may be classified into congenital, senile, diabetic, complicated cataract, etc. The factors that cause such a state are many and X-ray radiation is but one of these factors.

cataract has been treated by surgery and by drug therapy (such as the administration of eye-drops containing catalin or glutathione and of KI or vitamin C) but drug therapy has failings in the fundamental treatment of cataract.

The glutathione monoalkyl esters are alkyl esters of glutathione with respect to its glycin carboxyl group. It has been found that these compounds are useful as therapeutic agents for cataract, in particular for cataract induced by radioactive rays or X-ray radiation. Surprisingly the effect is markedly superior to that of glutathione-containing eye-drops.

Namely, the present invention relates to prophylactic and therapeutic agents for cataract comprising glutathione monoalkyl ester of general formula (I) or salts thereof as effective component. Further, the present invention relates to radiation-protecting agents comprising glutathione monoalkyl ester of general formula (I) or salts thereof as effective component.

In formula (I), the alkyl group R is preferably a straight or branched group of 1 to 10, most preferably 1 to 5 or 6, carbon atoms.

Specific examples thereof include methyl, ethyl, propyl, isopropyl, butyl, isobutyl, tert-butyl, pentyl, isopentyl, neopentyl, 1-methylbutyl, hexyl, isohexyl, 2-methylpentyl, 1-ethylbutyl, heptyl, octyl, nonyl and decyl groups. Examples of the salts thereof include inorganic salts such as hydrochloride, nitrate, sulfate, etc., and organic salts such as oxalate, p-toluenesulfonate, maleate, etc.

The glutathione monoisopropyl ester (isopropyl $\gamma$-L-glutamyl-L-cystenylglycinate) sulfate used in the following experiments is prepared as described in EP-A-0257992 or JP-A-62-09695.

Four-week old SLC Wistar rats are divided into three groups each of ten animals. The first group is a normal control group. The second group is an X-ray irradiated control group to which is administered intraperitoneally 1ml/kg of physiological saline solution three times a week after X-ray irradiation. For the third group, 20mg/kg of glutathione monoisopropyl ester sulfate is administered intraperitoneally three times a week after X-ray irradiation. X-ray irradiation is carried out by irradiating the cephalic portion of concious rats at 1,000 rad.

After X-ray irradiation, the lenses are monitored for opacity with a slit lamp every two weeks. The extent of cataract induced by X-ray radiation is scored using seven grades ranging from I to VII - from "clear" (namely, no change in the apparent view and substantially the same conditions as the normal control group) to "opaque". When the lenses of the irradiated control group (the second group) reaches level IV or more (at 28 weeks), the tests are finished. Then the lenses are extracted and measured for biochemical parameters [amounts of nonprotein-SH (Ellman's method), amounts of $Na^+/K^+$ and $Ca^{++}$ (Atomic absorption method)].

The results obtained with the slit lamp, are shown in Fig. 1. The therapeutic effects of glutathione monoisopropyl ester sulfate (the results of biochemical analysis) are shown in Fig. 2, (1) to (3).

From the results in which the V grade is greater than 50% in the X-ray irradiated control group (the second group) while the II group is greater than 50% in the glutathione monoisopropyl ester sulfate group (the third group, 20mg/kg), it is evident that the ester exhibits an outstanding inhibiting action.

Fig. 2 shows results of biochemical analysis of the glutathionemonoisopropyl ester sulfate group lenses. Figure 2 (1) shows the inhibiting action on the decrease of the amount of SH ; Figure 2 (2) shows the inhibiting action on the increase of the $Na^+/K^+$ level; and Figure 2 (3) shows the inhibiting action on the

increase of $Ca^{++}$ level.

From the foregoing experimental results, it is noted that pharmaceutical preparations containing as effective component compound of general formula (I) or salts thereof exhibit the action of significantly inhibiting the opacity of the lens of rats induced by X-ray radiation, upon intraperitoneally administering 20mg/kg three times a week for 28 weeks. Accordingly, pharmaceutical preparations containing compounds of general formula (I) or salts thereof as effective component are considered to be sufficiently effective in the prophylaxis of X-ray-induced cataract in humans. It is presumed that the mechanism of these anti-cataract effects results from the scavenger action of free radicals. Further, senile cataract in humans is histologically similar to X-ray induced cataract and results from the existence of free radicals, which is one of triggers inducing senile cataract. Thus, it can be said that pharmaceutical preparations containing compounds of general formula (I) or salts thereof as effective component are useful for the prophylaxis and treatment of senile cataract.

The glutathione monoalkyl ester or salts thereof can be administered in combination with conventional carriers, excipients and other additives, which are conventionally employed. They can be administered orally or parenterally. They are preferably administered parenterally in the form of injectable preparations, eye-drops, etc.

The dose is about 10mg to 1,600mg per person for an injectable preparation, and about 1 to 2% solution for eye-drops. Also the frequency of administration is commonly about one to three times daily and it is desirable that the preparation is formulated so as to comply with the above-mentioned conditions. The compounds of general formula (I) and salts thereof have acute toxicity ($LD_{50}$) of approximately 5.3g/kg in intra- peritoneal injection to mice (for example, in the case of the isopropyl ester).

To prepare one eye-drop formulation according to the invention, a table containing 100 mg of glutathione monoisopropyl ester sulfate was dissolved in a solution of 0.45 mg of benzalkonium chloride and 35 mg of boric acid, and the resulting mixture was shaken gently to obtain transparent, colorless eye-drop solution.

Opthalmic solutions, e.g. eye-drops, according to the invention will usually be a dilute (e.g. 1-2 wt.%) aqueous or aqueous alcohol solution of one or more compounds selected from the glutathione monoalkyl esters and their salts, and may also contain conventional opthalmic solution or eye-drop component(s) - e.g. boric acid and/or borax, antimicrobial agent (e.g. benzalkonium chloride) etc.

## Claims

1. The use, for the preparation of a medicament for the treatment or prevention of cataract, of compound selected from glutathione monoalkyl esters of the following formula and salts thereof

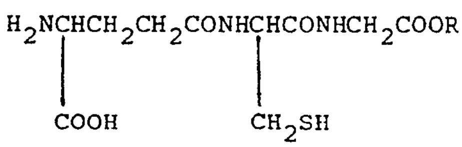

wherein R is an alkyl group.

2. The use, for the preparation of a radiation-protective agent, of compound selected from glutathione monoalkyl esters of the following formula and salts thereof

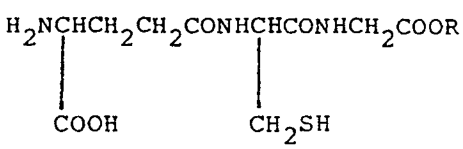

wherein R is an alkyl group.

3. A use according to claim 1 or 2 wherein R contains 1 to 10, preferably 1 to 6, carbon atoms.

4. A use according to claim 3 wherein R is isopropyl.

5. Eye-drops containing at least one compound selected from glutathione monoalkyl esters of the following formula and salts thereof

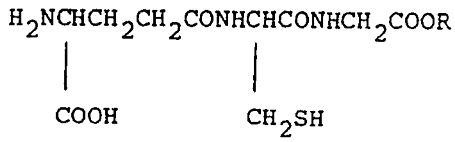

wherein R is alkyl.

6. Eye-drops according to claim 5 wherein R is $C_1$-$C_{10}$, preferably $C_1$-$C_5$.

7. Eye-drops according to claim 6 wherein R is isopropyl.

8. Eye-drops according to claim 5, 6 or 7 which are a dilute aqueous solution of said compound(s) and which optionally also contain boric acid and/or borax.

9. A prophylactic and therapeutic agent for cataract comprising as an effective component a glutathionemonoalkyl ester of the formula

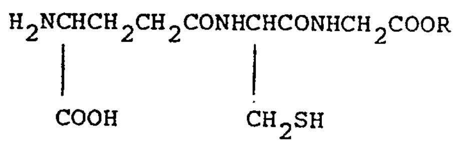

wherein R represents an alkyl group, or a salt thereof.

10. A radiation-protecting agent comprising as an effective component a glutathione monoalkyl ester of general formula

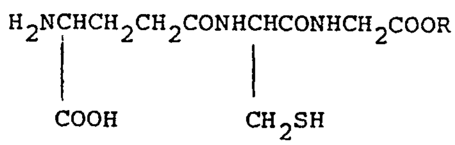

wherein R represents an alkyl group, or a salt thereof.

Fig. 1

| scoring | | X-ray irradiated control | glutathione monoisopropyl ester (20mg/kg) |
|---|---|---|---|
| I | 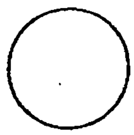 | — | 13% |
| II | 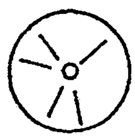 | — | 56% |
| III | 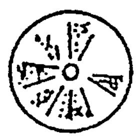 | — | 13% |
| IV | 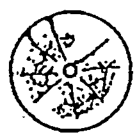 | 30% | 18% |
| V | 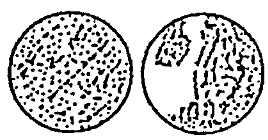 | 50% | — |
| VI | 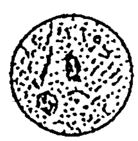 | 10% | — |
| VII | 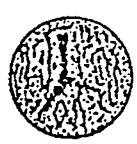 | 10% | — |

Fig. 2 (1)

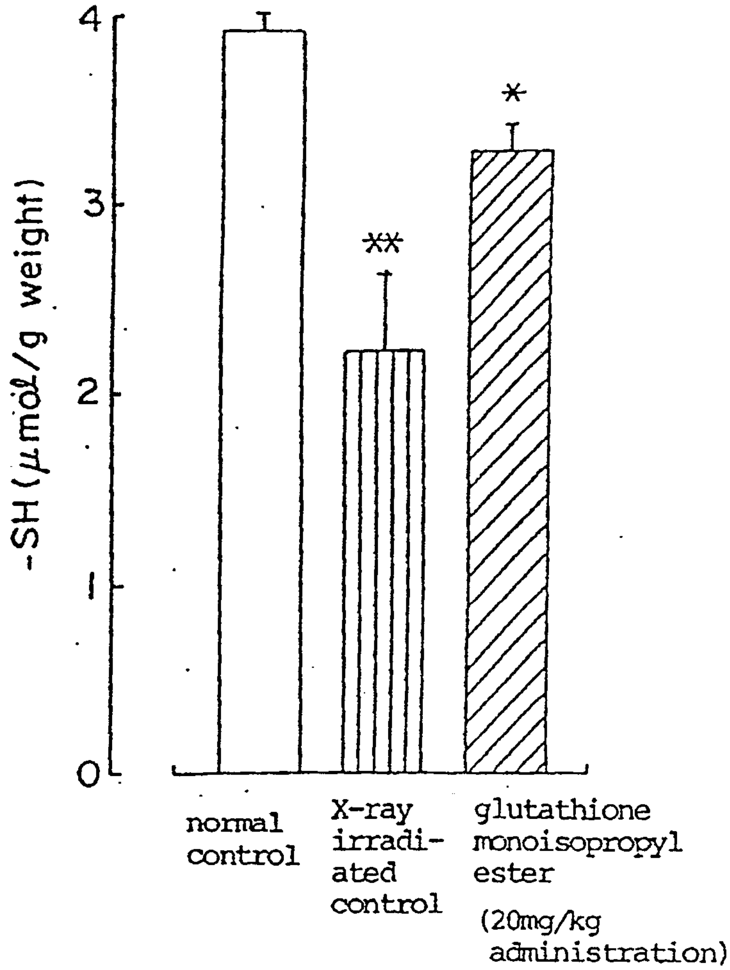


*P< 0.05 (vs normal control)
*P< 0.01 (vs normal control)

Fig. 2 (2)

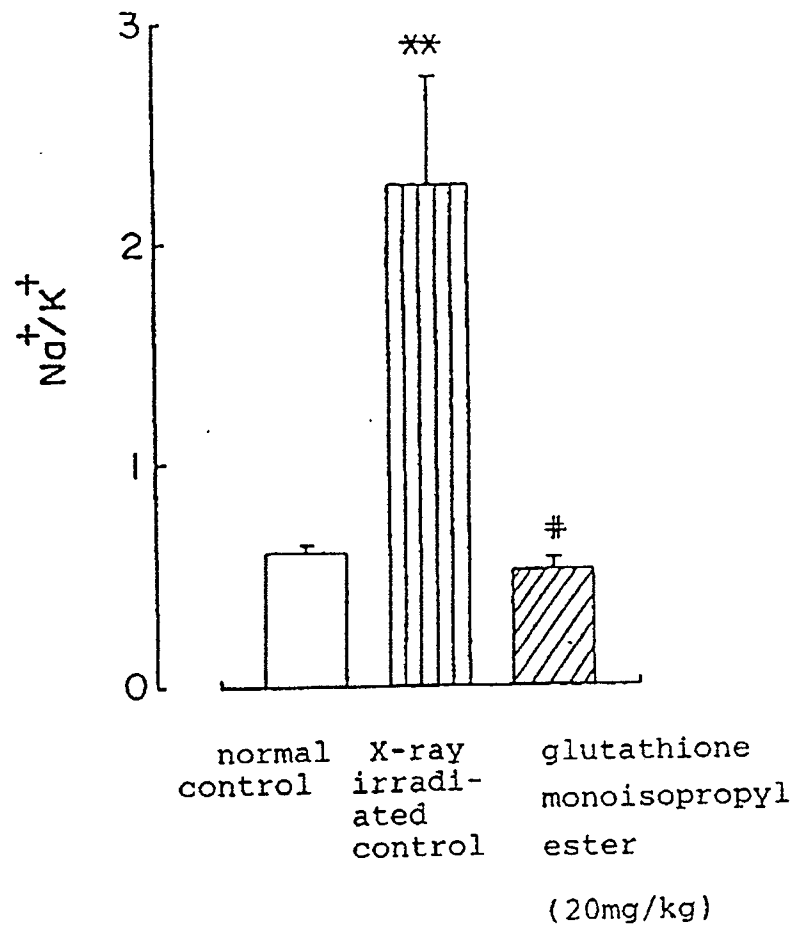


*P< 0.05(vs normal control)
**P< 0.01(vs normal control)
#P< 0.05(vs X-ray irradiated control)

Fig. 2 (3)

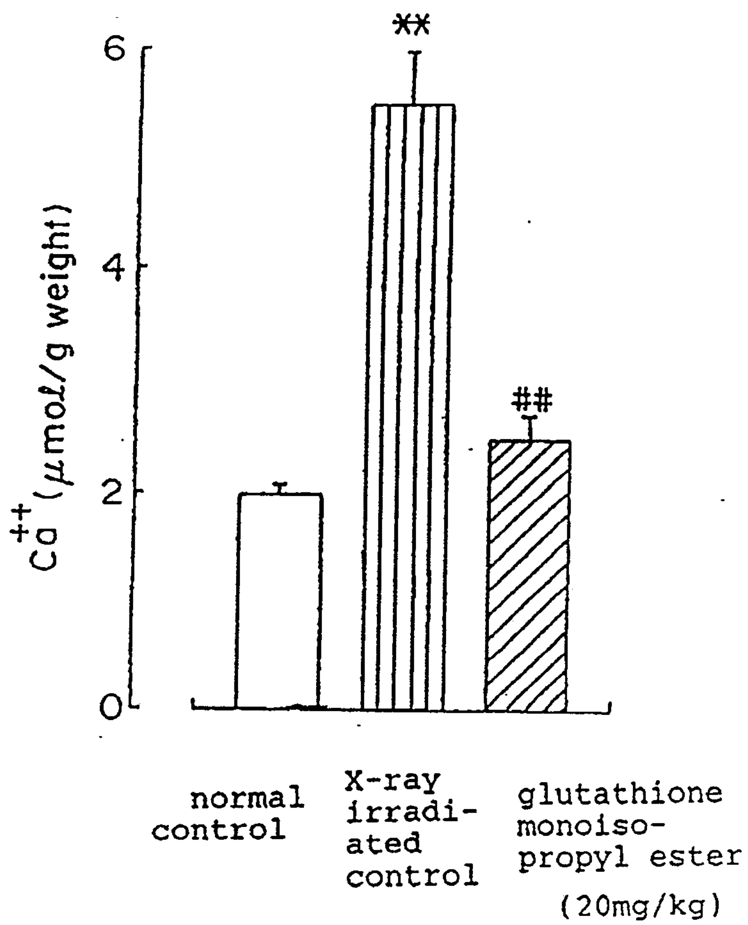


**P< 0.01(vs normal control)
##P< 0.01(vs X-ray irradiated control)